Europäisches Patentamt

European Patent Office

Office européen des brevets

(11) Publication number: **0 353 651**
**A1**

# EUROPEAN PATENT APPLICATION

(21) Application number: **89113981.8**

(22) Date of filing: **28.07.89**

(51) Int. Cl.⁴: **A61F 2/16 , G02B 1/04 ,**
**B29D 11/00**

(30) Priority: **04.08.88 CS 5469/88**

(43) Date of publication of application:
**07.02.90 Bulletin 90/06**

(84) Designated Contracting States:
**CH DE FR GB IT LI**

(71) Applicant: **CESKOSLOVENSKA AKADEMIE VED**
**Narodni 3**
**Praha 1(CS)**

(72) Inventor: **Wichterle, Itto**
**Na Andelce 27**
**Praha 6(CS)**
Inventor: **Michalek, Jiri, Dipl.-Ing.**
**5.kvetna 31**
**Praha 4(CS)**
Inventor: **Vacik, Jiri CSc, Dipl.-Ing.**
**Mechenická 18**
**Praha 4(CS)**

(74) Representative: **Patentanwälte Beetz sen. -**
**Beetz jun. Timpe - Siegfried -**
**Schmitt-Fumian- Mayr**
**Steinsdorfstrasse 10**
**D-8000 München 22(DE)**

(54) **Intraocular lens and methods for its preparation.**

(57) An intraocular lens consisting of a core of a polymer with a refractive index of 1.37 to 1.6 with the shape of a biconvex or plano-convex lens and a peripheral case covering one surface of the core either completely or only on its circumference and the other surface to a distance of atmost 2 mm from the core axis is produced by placing the core on a plane or rotationally symmetric curved casting surface, overcasting it with a monomer mixture in order to wet the lower surface of the core either completely or only in its circumferential part and the upper surface to a distance of atmost 2 mm from the core axis and then polymerizing the monomer mixture by thermal or photochemical polymerization.

FIG.1

## Intraocular lens and methods for its preparation

The invention pertains to an intraocular lens and methods for its production.

The methods used for the production of intraocular lenses so far mostly employ mechanical technologies both for shaping the central part with optical function and for providing this part with peripheral supports which should centre the lens in the anterior or posterior chamber of the eye. If the central optical part is made first independently of its periphery, the peripheral supports have to be attached additionally, for example, in such a way that shaped strings from polypropylene are fixed in holes drilled radially into the lens. The whole lens with its peripheral supports may also be made by mechanical working, but it is a complex operation with high demands particularly with respect to the required perfect smoothness of all surfaces. Without this smoothness, undesirable depositions of lipids, proteins and whole cells could occur when the lens is applied into the eye. It is obvious, that intraocular lenses cannot be manufactured by mechanical processing of soft and elastic materials, for example, slightly crosslinked poly-(butylmethacrylate). However, soft lenses are prospective mainly because they can be introduced into the eye in a coiled state through a relatively small incision.

It is the object of the invention to provide an intraocular lens which is free from the shortcomings related above and which enables the application and centering of the lens in the eye with a minimum of irritation of the living eye tissues, and methods for its preparation.

This object is achieved according to the claims. The dependent claims relate to preferred embodiments.

The intraocular lens according to the invention consists of a core of a polymer having a refractive index of 1.37 to 1.6 in the shape of a biconvex or plano-convex lens and a peripheral case, the case covering one surface of the core either entirely or only on the circumference of a planar or rotationally symmetric surface and the other surface up to a distance of at most 2 mm from the core axis in order not to interfere with the optical function of the lens.

The peripheral case may be shaped in very diverse ways. According to a preferred embodiment the peripheral case is a peripheral film with a thickness of 0.1 to 1 mm, which can be provided with holes any parts of which may be left out thus forming arbitrarily shaped supports connected with the lens.

The material of the peripheral case may be identical or very similar to the material of the built-in lens, the unit representing then a monolithic intraocular lens. However, the combination of a central part of a strongly refractive and easily workable material with a peripheral case of a soft material, which enables the application of the lens to the eye with a minimum irritation of the living tissues of the eye is particularly advantageous.

The core may be made of a hydrophilic material, advantageously of a crosslinked copolymer of 2-hydroxyethyl methacrylate, having, after equilibrium swelling with water, a refractive index of 1.40 to 1.45. The case may be also made of a more hydrophilic material than the core. Such a more hydrophilic material can be for example a crosslinked copolymer of 2-hydroxyethyl methacrylate (HEMA).

The crosslinked copolymer of 2-hydroxyethyl methacrylate may contain 0.2 - 2 mass-% methacrylic acid or acrylic acid in the form of an alkaline salt or 2-(2'-hydroxyethoxy)ethyl methacrylate (diethylene glycol methacrylate) in an amount of preferably 40 - 70 mass-%.

The intraocular lens according to the invention may be advantageously made by placing the core made of a polymeric optical material in the shape of a biconvex or plano-convex lens on a plane or rotationally symmetric curved casting surface and overcasting it with a monomer mixture in a sufficient amount for wetting the lower surface of the core either completely or only on its circumference and the upper surface to a distance of atmost 2 mm from the core axis and polymerizing the monomer mixture by thermal or photochemical polymerization.

A rotationally symmetric concave area is advantageously formed in the casting surface, into which the core fits so that its edge is on the level of the surrounding casting surface. Spreading of the monomer mixture on a base wettable with the monomeric mixture can be demarcated by printing with a film of a non-wetting polymer, preferably a silicon polymer.

If the central optical surface of the core should remain uncovered with the case on both sides, it is advantageous to form the base of a material which is highly viscous, for example beeswax, or which changes into a plastic state at a moderately increased temperature, for example, paraffin or beeswax. The core may be fixed to this base by gentle pressing into the base, thus protecting the lower surface of the core from wetting with the monomer mixture. It is particularly suitable to form a liquid base on a horizontal surface which determines the shape of the peripheral part of the case by sharp edges. If such a surface for a liquid base is also provided with drilled holes, the corresponding holes are also finally formed on the circumference of the case.

In another embodiment the case may be formed around the core by clamping the core between two parts of an elastic mold, which after clamping tightly adher to the central surfaces of the core with the optical function and, at the same time, leave an empty space having the required shape of the case around the edge of the core. This space is then filled with a monomer mixture containing a polymerization catalyst and which is polymerized by increasing the temperature or by irradiation.

In order to prevent the formation of possible inner stresses or the deformation of the core and the case, it is desirable that the swelling coefficient of the core with physiological saline differs from the swelling coefficient of the polymerized monomer mixture in the equilibrium with physiological saline by atmost 0.2 and preferably by less than 0.05.

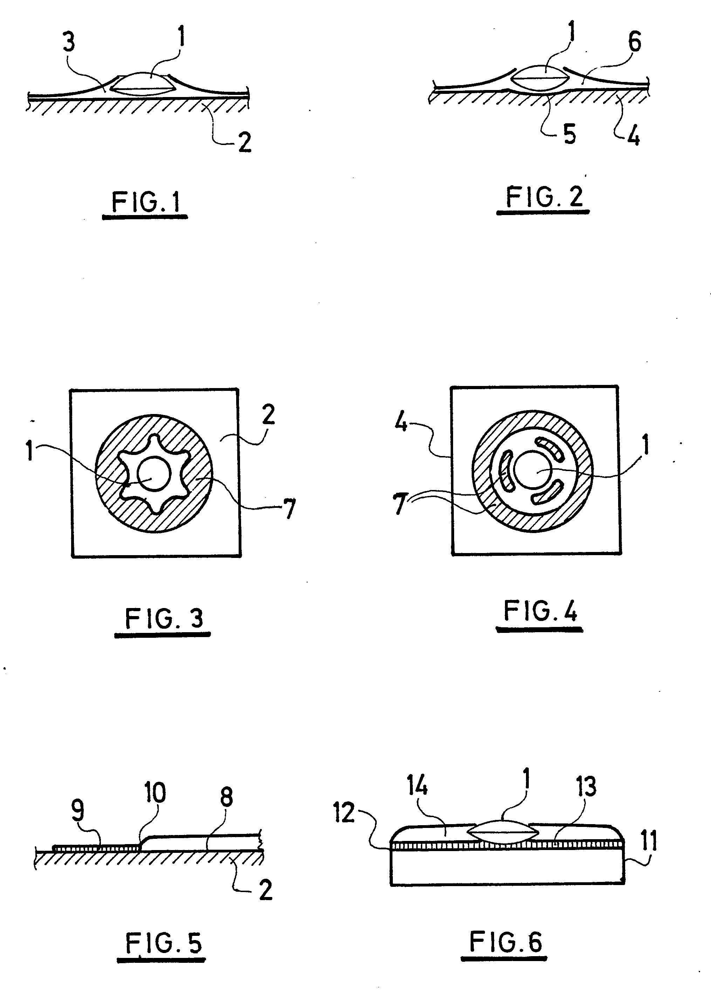

The invention is further elucidated by the drawings: Figures 1, 2, 5, 6, 8, 9, and 10 show the intraocular lens in the sectional view and Fig. 3, 4, and 7 in the view from above.

Fig. 1 shows an axial section of an intraocular lens according to the invention, when the casting surface is planar. The structure near the core is formed by capillary forces. The core 1 is placed on the base 2 and cast in the material of the peripheral case 3, thus forming a plano-concave lens which lowers the refraction of the core. The core of this embodiment must therefore be of a material with high refraction.

Fig. 2 shows a sectional view of a lens cast on the base 4 which is provided with a well 5 having optical quality and the shape of a spherical cap. The core is inserted into this well and cast with a monomer mixture 6. In this embodiment the refraction of core is minimally reduced since the core adhers to the convex-concave middle part of the peripheral case and the lens thus remains fitted by its edge approximately in the plane of the peripheral film.

Adaptation of the peripheral film of the core may be carried out by cutting or turning off parts of the cast smooth film or by punching or drilling holes. However, the edge of the case may be cast directly in any complicated shape. This method consists in forming a print of a non-wettable film on the base wettable with a monomer mixture, advantageously by means of a shaped stamp. For example, in casting a case of hydrophilic monomers, as HEMA, prints 7 of for example viscous silicon oil can be made in advance on the glass base 2 or 4 around the core, as it is shown in Fig. 3 or 4. Fig. 5 shows the magnified sectional view of a rounded edge 10 of the cast monomer mixture at the boundary between the wetted surface 8 of the base and the non-wetted surface of the print 9, which has without doubt a favourable effect on the soft resting of the support on the eye tissue.

Fig. 6 shows an embodiment of the procedure according to the invention in a sectional view. The core 1 of a dry hydrophilic material is fixed to the support 11 for a liquid base by means of a small amount of a highly viscous mineral oil or sugar syrup 13 which forms a sharp edge 12 demarcating a circular or diversely shaped circumference for the case. This structure is then overcast with a monomer mixture 14.

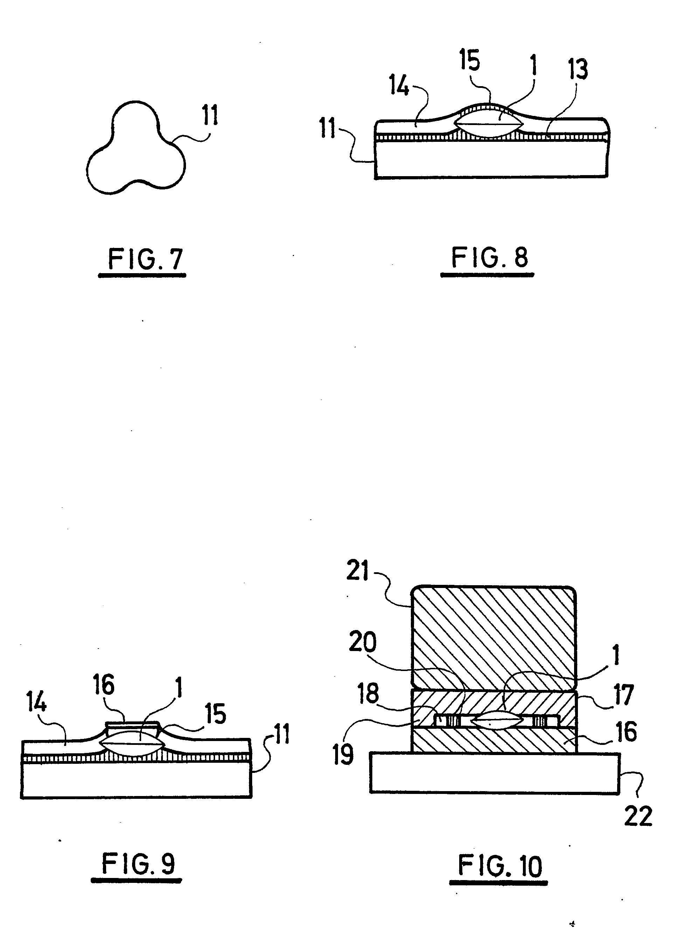

In Fig. 7 a complex shaped support 11 for a liquid base, which demarcates the supports of an intracameral lens, is viewed from above.

The sectional view in Fig. 8 shows a procedure similar to that of Fig. 6. The core 1 however is additionally protected on the upper middle part of the surface with a thin film 15 of a highly viscous material of the same kind as the liquid base 13. In this way the possible climbing of the poured monomer mixture 14 on the central surface of the core 1, which could damage its optical quality, is avoided.

In Fig. 9 the precedure of Fig. 8 is supplemented by securing the upper protective film 15 against the possible dripping down or even complete flooding of the core 1 by a disk of a strong film 16, which is wetted with the protective liquid film 15. This film is then safely held by capillary forces in the center of core.

Fig. 10 shows the casting of a hydrophylic case around the core 1 clamped between two parts of a mold of an unfilled transparent silicon rubber. The lower part 16 of the mold has in the relaxed state the shape of a flat cylinder into the upper surface of which the lower surface of the core 1 is pressed after having been clamped at the edges. Similarly the upper surface of the core 1 is pressed into the lower central surface of the upper part 17 of the mold. A free space 18 formed by the elevated peripheral edge 19 of the part 17 having inside the shape of the required peripheral curve of the case or also the projections 20 determining the desirable holes in the case remains between both parts 16 and 17 of the mold. The optimal clamping of the core is provided with a weight 21, which pushes both parts of the mold against the solid base 22. After assembling all parts, the space 18 is filled, e.g., by means of an inserted bent hypodermic needle, with a monomer mixture, which is then brought to polymerization conditions.

The outline of the prepared intraocular lens corresponds to the non-hatched area in the center of the hatched area in Figs. 3 and 4.

The core 1 may be manufactured by mechanical processing of an optical polymeric material, for example, poly(methylmethacrylate), or by casting a monomer mixture in concave molds with a free surface according to CS-B-245,361 (CA-B-1,232,406), or by polymerizing a drop of the monomer mixture resting on a liquid base according to CS-patent application 4955-88. Particularly the cores made by monomer casting are well suited for further adaption according to this invention because they have a perfectly sharp edge which well fits into a case.

The invention is further illustrated in the examples.

## Example 1

A biconvex lens made of the crosslinked copolymer of 2-hydroxyethyl methacrylate according to CS-patent application 4955-88, having a weight of 22 mg, a diameter of 4.9 mm, and a refractive index of 1.52, was placed on the planar surface of a mold of nonpigmented polypropylene. The surface of the mold was circular with a diameter of 12.9 mm demarcated by an elevated edge. A monomer mixture containing 99.5 mass-% 2-hydroxyethyl methacrylate and 0.5 mass-% ethylene glycol dimethacrylate with 0.5 mass-% benzoin ethyl ether (further called "photoinitiator"), calculated on the monomer mixture, was dripped with a micropipette on this surface as long as the edge of the lens and the mold were wetted with a uniform layer of the mixture. The mold was then transferred into an inert atmosphere of nitrogen and irradiated with UV rays of a mercury lamp RVK 125 W for 20 minutes. The mold was then submerged in distilled water in order to swell the polymerized lens which was then measured in the equilibrium swollen state. The obtained hydrogel intraocular lens had an optical core with a diameter of 5.85 mm and a peripheral film with a circular shape with a diameter of 15.5 mm and a thickness of 0.6 mm. The refractive index of the core and case was 1.435.

## Example 2

A biconvex lens made of the crosslinked copolymer of 2-hydroxyethyl methacrylate having a weight of 22 mg and a diameter of 4.9 mm was inserted into a common polypropylene spin-casting mold for contact lenses. Then, 30 mg of a monomer mixture consisting of 35.78 mass-% 2-hydroxyethyl methacrylate, 43.72 mass-% diethylene glycol monomethacrylate, 20 mass-% glycerol and 0.5 mass-% ethylene glycol dimethacrylate with 0.5 mass-% photoinitiator, related to the monomer mixture, was dosed into the mold. The charged mold was transferred into a vertical column rotating at 365 r.p.m. where polymerization of the monomer mixture initiated by UV radiation proceeded for 15 min in an inert atmosphere of nitrogen. The intraocular lens was released from the mold by swelling with water and brought into equilibrium with distilled water. 10 holes were cut in its peripheral part in 2 mm distance from the circumference by an adapted punch with circular section and a diameter of 1.2 mm. The resulting intraocular lens had a total diameter of 14.1 mm, an optical core with a diameter of 5.85 mm, a thickness of the peripheral film case of 0.05 mm in 1 mm distance from the circumference, and 0.35 mm in 1 mm distance from the edge of the core. The refractive index of the core and case was 1.435 respectively 0.11 MPa. The curvature of the rotationally cast surface of the case may be approximately described by the radius of the corresponding spherical surface, which was in this case 9 mm.

## Example 3

A print of silicone oil was formed by a rubber stamp on a perfectly degreased glass base in such a way that it demarcated an area in the form of a broad letter X with rounded tops, the longest dimension of which was 11.25 mm. A biconcave lens of the same parameters as in example 1 was placed in the center of this area. A thin film of a monomer mixture with a composition according to the example 1 was then formed on the demarcated area "X" by polymerizing the mixture. The resulting intraocular lens was submerged in distilled water as in example 1. The lens consisted of an optical core with a diameter of 5.95 mm in the swollen state and four elastic supports. The longest dimension of the whole intraocular lens in the swollen state was 13.5 mm.

## Example 4

A biconvex lens made of the polymer of ethylene glycol dimethacrylate having a weight of 22 mg and a diameter of 6.66 mm was placed on a concave glass surface with a radius of 9.5 mm demarcated by a diamater of 10.5 mm. A monomer mixture containing 59.5 mass-% 2-hydroxyethyl methacrylate, 0.5 mass-% ethylene glycol dimethacrylate, and 40 mass-% diacetine was dosed onto this surface in an amount of 50 mg. The mixture was polymerized by radical polymerization. The resulting intraocular lens consisted, after swelling with physiologic saline into equilibrium in the given medium, of a virtually nonswelling core with a diameter of 6.6 mm and K = 1 and a case with a swelling capacity of 38 mass-%, K = 0.98, a diameter of 10.25 mm and a thickness of 0.3 mm at a diameter of 8.4 mm (K is the coefficient of expansion).

## Example 5

The shaped piece shown in Fig. 7 was milled from an aluminum sheet with a thickness of 1 mm as a support 11 for the liquid base, while sharp edges 12 were left on its circumference. The largest and smallest diameter of this support was 12 respectively 8 mm. The liquid base consisting of a mixture of beeswax and common concentrated detergent in the ratio 5:1 was then dripped in an amount of 0.3g on the support. After the base uniformly spread over the whole surface of the support, it was heated for 30 minutes on a plate to 80 °C in order to acquire a proper thickness by partial drying. The same prefabricated lens from poly-HEMA as that described in example 1 was placed in the center and the base was overcast with 0.4g of the monomer mixture according to example 1. After 30 minutes of irradiation with UV light in an atmosphere of carbon dioxide, the intraocular lens was swollen with water, removed from the base, washed with distilled water for several days, placed into physiological saline, and sterilized in an autoclave at 140 °C.

## Claims

1. Intraocular lens consisting of
- a core (1) of a polymer having a refractive index of 1.37 to 1.6 in the shape of a biconvex or plano-convex lens and
- a peripheral case (3)
the case covering one surface of the core (1) entirely or only on the circumference of a planar or rotationally symmetric surface and the other surface up to a distance of atmost 2 mm from the core axis.

2. The intraocular lens according to claim 1, wherein the case (3) is a peripheral film with a thickness of 0.1 to 1 mm.

3. The intraocular lens according to claim 1 or 2, wherein holes are provided in the peripheral film.

4. The intraocular lens according to claim 1 or 2, wherein only parts forming the supports of the intraocular lens are left of the peripheral film.

5. The intraocular lens according to any one of claims 1 to 4, wherein the case (3) and the core (1) are made of the same material.

6. The intraocular lens according to any one of claims 1 to 5, wherein the core (1) is made of a hydrophilic material having, after equilibrium swelling in water, a refractive index of 1.40 to 1.45.

7. The intraocular lens according to claim 6, wherein the hydrophilic material is a crosslinked copolymer of 2-hydroxyethyl methacrylate.

8. The intraocular lens according to claim 1 to 4, wherein the case (3) is made of a more hydrophilic material than the core (1).

9. The intraocular lens according to claim 8, wherein the more hydrophilic material is a crosslinked copolymer of 2-hydroxyethyl methacrylate.

10. The intraocular lens according to claim 9, wherein the crosslinked copolymer of 2-hydroxyethyl methacrylate contains 0.2 - 2 mass-% methacrylic acid or acrylic acid in the form of an alkaline salt.

11. The intraocular lens according to claim 9, wherein the crosslinked copolymer of 2-hydroxyethyl methacrylate contains 2-(2'-hydroxyethoxy)-ethylmethacrylate (diethyleneglycolmethacrylate) in an amount of preferably 40 -70 mass-%.

12. Method for producing the intraocular lens according to any one of claims 1 to 11, characterized in that the core made of a polymeric optical material in the form of a biconvex or plano-convex lens is placed on a plane or rotationally symmetric curved casting surface and over cast with a monomer mixture in a sufficient amount for wetting the lower surface of the core either entirely or only on its circumference and the upper surface of the core to a distance of atmost 2 mm from the core axis, and the monomer mixture is polymerized by thermal or photochemical polymerization.

13. The method according to claim 12, characterized in that the center of the casting surface is protected against wetting with the monomer mixture by a covering layer immiscible with the monomer mixture.

14. The method according to claims 12 and 13, characterized in that the casting surface has a rotationally symmetric concave surface into which the core fits so that its edge is on the level of the surrounding casting surface.

15. The method according to claims 12 and 13, wherein the spreading of the monomer mixture on the casting surface is limited by a print of a film of a non-wetting polymer, preferably a silicon polymer.

16. The method according to claims 12 and 13, wherein the polymerization of the monomer mixture is carried out under rotation on a concave casting surface.

17. The method according to any one of claims 12 to 16, wherein the central part of the upper surface of the core is coated with a layer immiscible with the monomer mixture.

18. The method for producing the intraocular lens according to any one of claims 1 to 11, characterized in that the core of a polymeric optical material having the form of a biconvex or plano-convex lens is clamped between two parts of a mold of elastic material provided with a recess leaving after clamping a free space having the shape of a peripheral case around the edge of the clamped lens, this space is completely filled with a monomer mixture, which is then polymerized by thermal or photochemical polymerization.

19. The method according to any one of claims 12 to 18, characterized in that the swelling coefficient of the core with physiological saline differs from the swelling coefficient of the polymerized monomer mixture brought into equilibrium with physiological saline by atmost 0.2, preferably by atmost 0.05.

FIG.1

FIG. 2

FIG. 3

FIG. 4

FIG. 5

FIG.6

FIG. 7

FIG. 8

FIG. 9

FIG. 10

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | <u>DE - A1 - 2 725 219</u> (TITMUS EUROCON KONTAKT-LINSEN KG) <br> * Claims 1,8; fig. * | 1,2,4, 5 | A 61 F 2/16 <br> G 02 B 1/04 <br> B 29 D 11/00 |
| A | <u>DE - A1 - 3 610 833</u> (INPROHOLD ESTABLISHMENT) <br> * Column 3, lines 37-49,67 - column 4, line 6; fig. 1,2 * | 1-4 | |
| A | <u>GB - A - 2 124 500</u> (MAZZOCCO) <br> * Page 3, lines 84-100; page 4, lines 53-95; fig. 2,3 * | 1,2,5 | |
| A | <u>US - A - 4 704 123</u> (SMITH) <br> * Claim 1; fig. 1-6 * | 1 | |
| A | PATENT ABSTRACTS OF JAPAN, unexamined applications, P field, vol. 4, no. 115, August 16, 1980 THE PATENT OFFICE JAPANESE GOVERNMENT page 6 P 23 <br> * Kokai-Nr. 55-69 101 (MITSUBISHI RAYON K.K.) * | 18 | |

TECHNICAL FIELDS
SEARCHED (Int. Cl.4)

A 61 F
G 02 B
B 29 D

The present search report has been drawn up for all claims

| Place of search <br> VIENNA | Date of compilation of the search <br> 10-11-1989 | Examiner <br> HAUSWIRTH |
|---|---|---|